# EUROPEAN PATENT SPECIFICATION

(11) **EP 3 790 530 B1**
(45) Date of publication and mention of the grant of the patent: **06.07.2022**
(21) Application number: 19729827.6
(22) Date of filing: 07.05.2019
(51) Int. Cl.: A61K 8/58, A61K 8/73, A61Q 19/00, A61K 8/04

(54) **MEDICAL OR COSMETIC COMPOUNDS, AND COMPOSITION THUS OBTAINED**
MEDIZINISCHE ODER KOSMETISCHE VERBINDUNGEN UND SO ERHALTENE ZUSAMMENSETZUNG
COMPOSÉS MÉDICAUX OU COSMÉTIQUES, ET COMPOSITION AINSI OBTENUE

(30) Priority: 07.05.2018 IT 201800005118
(43) Date of publication of application: 17.03.2021
(73) Proprietor: KLV GROUP LTD, London SW11 4FA (GB)
(72) Inventor: SIGNORETTO, Michela, 30175 Venezia (IT); MENEGAZZO, Federica, 30135 Venezia (IT); GHEDINI, Elena, 31021 Mogliano Veneto (IT)
(74) Representative: Barker Brettell LLP
(86) International application number: PCT/IB2019/053710
(87) International publication number: WO 2019/215592

(56) References cited:
- WO-A1-94/07947
- THONGTHAI WITOON ET AL: "Effect of pH and chitosan concentration on precipitation and morphology of hierarchical porous silica", JOURNAL OF NON-CRYSTALLINE SOLIDS, vol. 357, no. 19, 30 June 2011 (2011-06-30), pages 3513-3519, XP028254218, ISSN: 0022-3093, DOI: 10.1016/J.JNONCRYSOL.2011.06.029 [retrieved on 2011-06-30] cited in the application
- YEH ET AL: "Synthesis and properties of chitosan/SiO"2 hybrid materials", MATERIALS LET, ELSEVIER, AMSTERDAM, NL, vol. 61, no. 6, 2 February 2007 (2007-02-02), pages 1292-1295, XP005870504, ISSN: 0167-577X, DOI: 10.1016/J.MATLET.2006.07.016 cited in the application

## Description

### FIELD OF THE INVENTION

The present invention relates to a process for the production of an organic-inorganic hybrid gel containing one or more medical and/or cosmetic compounds, capable of controlled release of said compounds when brought into contact with the skin.

### STATE OF THE ART

Compositions consisting of one or more active substances dispersed in a fluid vehicle that performs various functions including, for example, allowing a homogeneous and effective distribution of the active substances on the interested part and diluting the active substances to bring their concentration to values suitable for the desired purposes, are widely used in the cosmetic and dermatological field. A very wide range of these compositions exists in the field, which differ for example in the nature of the vehicle for active substances, or in the form in which said substances are loaded in the vehicle; both these characteristics, the nature of the vehicle and the method for the introduction of active substances, influence the release kinetics of active substances.

One class of such compositions is formed by an organic-inorganic hybrid vehicle, in which the organic component is in the form of nanoparticles, such as proteins or micellar systems, or in the form of systems leading to the formation of three-dimensional networks very similar to those present in polymeric systems, while the inorganic component may be used in many forms, for example in the form of particles dispersed in the organic matrix.

The production and characterization of mixed organic-inorganic systems are the object of various publications, such as the article "Synthesis and properties of chitosan/SiO2 hybrid materials", J.-T. Yeh et al., Materials Letters 61 (2007) 1292-1295; the article "Effect of pH and chitosan concentration on precipitation and morphology of hierarchical porous silica", T. Witoon et al., Journal of Non-Crystalline Solids 357 (2011) 3513-3519; and the patent application WO 94/07947 A1.

The article "Redox and pH dual-responsive PEG and chitosan-conjugated hollow mesoporous silica for controlled drug release", J. Jiao et al., Materials Science and Engineering C 67 (2016) 26-33, describes a system for the controlled release of a drug (the antibiotic doxorubicin). The system consists of porous silica nanospheres having a diameter of about 250 nm, in which the antibiotic is loaded. Chitosan, a naturally derived polymer, is chemically bound to the surface of the nanospheres and is in turn bound to polyethylene glycol (PEG); these polymers control the nanosphere dispersibility in water and initially form a barrier to drug release but, depending on the pH and redox potential of the surface contacted with, the polymeric barrier on the nanospheres surface can degrade allowing the release of the drug. The system described in this article is, however, very complex to realize, requiring a preventive phase of production of the nanospheres, then loading the same with the active substance, and finally a double functionalization step of the nanospheres surface with two different polymers.

The patent application US 2004/0247895 A1 describes a system comprising a matrix consisting of a silica-based gel, inside which there are molecules, in particular cyclodextrins, capable of hosting and then releasing active molecules. This system is produced by pre-loading the cyclodextrins with the active substance, then adding the charged cyclodextrins into a solution of silica gel precursors and forming the gel through a sol-gel process. Also in this case, the process requires at least two clearly distinct phases, the first of cyclodextrin loading and the second of formation of the gel matrix containing them. In addition to this complex preparation, while it may have good rheological properties depending on its formulation, a silica gel is still poorly biodegradable.

Patent application WO 2012/038880 A2 describes compositions for cosmetic or dermatological use prepared by using the sol-gel technique and obtained by mixing: a component (a), consisting of at least one alkylalkoxysilane of formula R1ₓSi(OR2)₍₄₋ₓ₎ or [R1_{y}SiO_{(4-y)/2}]ₙ[R1ₓ(OR2)_{z}SiO_{(4-x-z)/2}]ₘ; (b) at least one monomer or oligomer of the alkoxysilane type of formula R3ₓSi(OR2)₍₄₋ₓ₎ or [R3_{y}SiO_{(4-y)/2}]ₙ[R3ₓ(OR2)_{z}SiO_{(4-x-z)/2}]ₘ; (c) at least one acid in an amount such as to bring the pH of the system to a value of between 3 and 6; and (d) water in sufficient amount to cause hydrolysis of the system. The system of this patent requires the use of complex components which are difficult to find commercially; moreover, also in this case, the composition is exclusively based on silane compounds and, therefore, poorly biodegradable; furthermore, the preparation method and the formulation of this patent are optimized to form a protective film on keratin-based tissues very quickly, once applied (the invention is mainly directed to specific cosmetic treatments related to make-up, lip gloss, eye shadows, nail polishes, etc.), and to maximize its chemical and mechanical resistance, and not in view of active components release.

Finally, patent US 6,352,699 B1 describes a cosmetic or dermatological composition capable of reacting and crosslinking when in contact with the skin, formed by (a) at least one organometallic compound, (b) at least one functionalized organic polymer or a precursor thereof, or at least one functionalized silicone or a precursor thereof (in the latter case, different from component (a)), (c) water and, optionally, (d) at least one alcohol. Also for this patent, the considerations made for the previous document are true, *i.e.* the fact that the materials described therein are poorly biodegradable, and directed to obtain stable films on the skin or other keratin tissues (nails, hair), but not to the release of active substances.

In addition to the aforementioned limits, the processes and products of the prior art use components that are almost entirely synthetically derived, while it would generally be preferable to have processes and products available in which, at least for the most part, naturally occurring or recycled raw materials are used, such as by-products or waste from other industrial productions, such as biomass; moreover, the compositions of the prior art generally contain surfactants to compatibilize the various components, but this may result in reduced compliance by end users of the product.

The object of the present invention is to provide an organic-inorganic hybrid gel which is simpler to produce and/or uses starting components which are easier to find than those of the prior art; in particular, the object of the invention is to provide an organic-inorganic hybrid gel which, in addition to using raw materials of natural origin or deriving from recycling, is also able to control the release of active substances in order to reduce the number of administrations, and therefore make the use thereof easier. Another object of the invention is to provide a process for the production of these compositions which allows to obtain, in a few steps, the final formulation, in the ready-to-use form, able to maximize bioavailability of the active components by releasing them in a controlled manner over time.

### SUMMARY OF THE INVENTION

This and other objects are achieved by the present invention, which in a first aspect thereof relates to a process for the preparation of a hybrid gel with three-dimensional network structure consisting of inorganic components and naturally derived organic components, said gel containing one or more medical and/or cosmetic compounds, comprising the following steps:
a) in a first container, forming an aqueous or hydroalcoholic suspension containing between 1 and 4% by weight of at least one polysaccharide and between 0.5 and 1.5 by weight of at least one regulator of rheological properties;
b) in a second container, suspending in water an alkoxide or an alkylalkoxide of silicon, titanium or mixtures thereof, and optionally precursors of oxides of one or more metals selected from zirconium, aluminum, and zinc, in a total amount of between 50 and 80% by weight, and adding to the suspension thus obtained a hydrolysis catalyst in a molar ratio ranging from 0.001 to 0.01 with respect to the total moles of alkoxide or alkylalkoxide, obtaining a solution;
c) adding one or more medical and/or cosmetic compounds to the suspension obtained in step a) if said one or more compounds are lipophilic and/or to the solution obtained in step b) if said one or more compounds are hydrophilic, in an amount such that said medical and/or cosmetic compounds are from 0.2 to 5% by weight of the total weight of said suspension and solution;
d) combining the suspensions and solutions obtained in steps a), b) and c), and maintaining the resulting suspension under stirring for a time varying between 10 minutes and 1 hour, obtaining a gel;
e) allowing the gel obtained in step d) to rest in a closed container for a time of at least 10 days and bringing the pH of the same to a value between 4.3 and 5.5.

In a preferred embodiment thereof, the process of the invention further comprises a further step d'), carried out between steps d) and e), which consists in adding a wetting agent to the gel obtained in step d).

In a second aspect thereof, the invention relates to a composition obtained by the process described above, in the form of a gel comprising (as percentages by weight):
- water in an amount of 65 to 85%;
- polysaccharides in an amount of 1 to 3%;
- regulators of rheological properties in an amount of 0.5 to 1.5%;
- SiO₂, TiO₂ or mixtures thereof, and optionally ZrO₂, Al₂O₃ and ZnO, in a total amount of 3 to 6%;
- one or more alcohols in an amount of 5 to 10%;
- medical and/or cosmetic compounds in an amount of 0.2 to 5%;
- salts, derived from neutralization of acids and bases used in the process, in an amount of 0.05 to 0.1%.

If the process is carried out in the preferred embodiment described above, said composition further contains at least one wetting agent in an amount of 3 to 5% by weight.

### BRIEF DESCRIPTION OF THE FIGURES

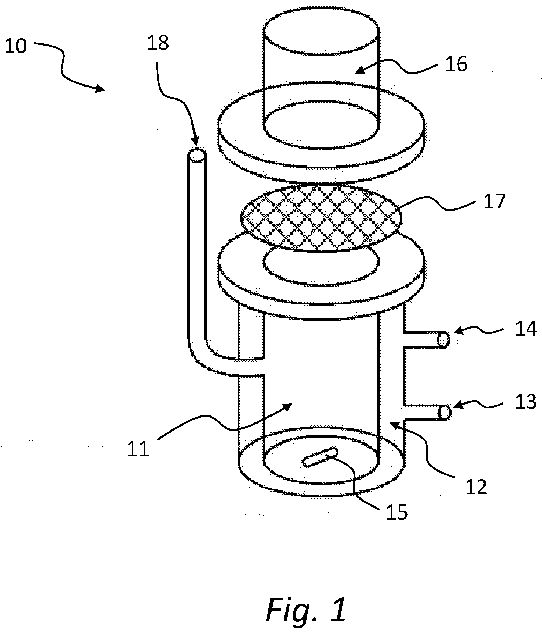

Fig. 1 shows in a schematic form a cell for measuring the release kinetics of medical and/or cosmetic compounds by compositions of the invention.

### DETAILED DESCRIPTION OF THE INVENTION

The invention will be described below with reference to the single figure. In the rest of the description, the terms "active ingredients" will be used to denote the medical and/or cosmetic compounds released by the composition, and "matrix" to denote the vehicle of the active ingredients, *i.e.* everything that forms a composition of the invention except the active ingredients.

In the first aspect thereof, the invention relates to a process for the production of an organic-inorganic hybrid gel for the release of active ingredients, comprising the steps a) to e) described above.

The first step of the process, a), consists in the preparation, in a first container, of an aqueous or hydroalcoholic suspension containing between 1 and 4% by weight of at least one polysaccharide and between 0.5 and 1.5% by weight of at least one regulator of rheological properties. Preferably, this suspension is exclusively water-based, without the addition of alcohols.

The polysaccharide may be selected from chitosan, pectin, carrageenan, xyloglucan, an alginate (for example, sodium alginate), a starch, and mixtures thereof. All these compounds are of natural origin and are easily commercially available, because they are widely used, for example, in the food industry. In particular, chitosan is obtained by deacetylation (generally in basic conditions) of chitin, a material forming the exoskeleton of insects, marine crustaceans, and the like; pectin is obtained by water extraction from fruit, especially citrus fruits or apples, followed by selective precipitation carried out with alcohols and salts; carrageenan is obtained by boiling red algae typical of the North Atlantic coasts; xyloglucan is mainly extracted from tamarind seeds; sodium alginate is extracted from cell walls of algae; while starch is extracted especially from potatoes and cereals.

The molecular weight of these polysaccharides may vary within wide limits, and it is often different, for the same type of polysaccharide, depending on the natural source from which it is extracted; for example, in the case of pectin, the molecular weight varies from 40 kDa to 150 kDa depending on whether it derives from apples or citrus fruits. This molecular weight is generally determined by viscosimetric method (extrapolation at zero concentration of the viscosity of solutions with different polymer concentration). The viscosimetric molecular weight is the value generally used in the technical sheets by suppliers of raw materials for pharmaceutical and cosmetic applications. Characteristic values of molecular weights for the cited polysaccharides are: carrageenan from 100 to 500 kDa; xyloglucan about 600 kDa; sodium alginate from 10 to 600 kDa; starch about 200 kDa.

For the purposes of the invention, the preferred polysaccharides are chitosan and pectin, in (commercially available) fractions having a molecular weight ranging from about 80 to 200 kDa in the case of chitosan, and about 100 kDa in the case of pectin. If the polysaccharide used is chitosan, or a mixture containing it, an acidic pH is required for the solubilization of this component, which is reached by adding one or more carboxylic acids. Some carboxylic acids are among the possible active ingredients of the gels of the invention, and as such may be used, alone or in synergy with other active ingredients (as detailed below); if the gel comprises one of these acids, this may be added to the suspension containing chitosan and be sufficient for the solubilization of the latter; this has been verified by the inventors, particularly in the case of glycolic acid. If, instead, the final gel does not involve the use of a carboxylic acid as an active ingredient, or the acidity it imparts is not sufficient, a carboxylic acid is added *ad hoc* to obtain complete solubilization of chitosan; in this case, the acid added as a solubilizer is preferably acetic acid, which is preferably used in the form of an aqueous solution at a concentration of between 1 and 3% by volume (v/v).

The regulator of rheological properties is selected from gum arabic, tragacanth gum, alginic acid and, preferably, guar gum, xanthan gum, and mixtures thereof in any ratio; these components are also widely used in the food industry and commonly commercially available.

The second step of the process, b), consists in the preparation, in a second container, of an aqueous or hydroalcoholic solution of an alkoxide or an alkylalkoxide of silicon, titanium or mixtures thereof, to which precursors of oxides of one or more metals selected from zirconium, aluminum and zinc may be optionally added; the total amount of metallic precursors (being silicon also included in the metals) is of between 50 and 80% by weight with respect to this solution; also in this case, the solution is preferably prepared only with water. The alkoxides or alkylalkoxides used as Si and Ti precursors are compounds of general formula:

Rₓ-M-(OR')₄₋ₓ

wherein M is a metal selected from Si and Ti, R and R', equal or different, are C2-C4 alkyl radicals, and x is 0 or 1. For the purposes of the present invention, preferred alkoxides or alkylalkoxides are tetraethoxysilane, Si(OCH₂CH₃)₄, generally referred to in the industry as TEOS, methyltriethoxysilane, CH₃-Si(OCH₂CH₃)₃, known as MTES, and titanium tetraisopropoxide, Ti(OCH(CH₃)₂)₄.

Zirconium, aluminum and zinc oxides precursors that may optionally be added in this process step are selected from aluminum isopropoxide, zirconium isopropoxide, zirconium butoxide, zinc sulfate, zinc acetate, and zinc acetylacetonate.

If the solution is prepared exclusively, or with a major proportion, of silicon alkoxides or alkylalkoxides, an acid, for example HCI, is preferably added thereto until a concentration of 0.1 M is reached in the solution, to accelerate the kinetics of hydrolysis and subsequent gelation of said compounds; the solution is maintained under stirring, for example by mechanical stirring, for a time varying between 10 minutes and one hour. On the other hand, if the only or main alkoxide present in the solution is a titanium compound, the use of HCI is not required, and a retarder of the gelation reaction, such as for example acetic acid, is added to the solution.

As it will be immediately clear, the two previous steps were named a) and b) for clarity of exposition, but this does not imply any temporal sequence required in performing them, and step b) may be performed before, after o simultaneously with step a).

Once the suspension and solution described above are prepared, the active ingredients of the final composition are added to one or both of them in step c). In particular, as said, lipophilic active ingredients are added to the suspension containing the polysaccharides and rheological regulators, while hydrophilic active ingredients are added to the solution prepared starting from the alkoxides or alkylalkoxides.

Active ingredients that may be added to said suspensions and solutions are the most varied of those known with cosmetic or medical dermatological action. To give only a few examples, among these active ingredients we can mention some carboxylic acids such as glycolic acid, mandelic acid, gallic acid, azelaic acid, ferulic acid and lipoic acid; caffeine; vitamin C and vitamin E (in particular, the compound δ-tocopherol); proanthocyanidins (extracted from red grape waste), polyphenols (extracted from citrus fruit peels and acorns), in particular resveratrol; and ellagic acid, the dilactone of hexahydroxydiphenic acid.

The gels of the invention may contain multiple active ingredients, for example mixtures of carboxylic acids and caffeine, resulting in a combination having synergistic effects.

These compounds have different actions, with an overall anti-aging effect; for example, acids and polyphenols have an antioxidant action, while other compounds among those mentioned counteract the decomposition of collagen, repair free radicals damage, and act as a natural sunscreen.

Methods for the addition of active ingredients are different depending on the specific compound and its physical form (for example, if it is a solid or a liquid); for example, in the case of carboxylic acids, these are added in the form of an aqueous solution, at a concentration of between 5 and 15% v/v, preferably between 6 and 12%. After adding the active ingredients to a suspension or solution prepared in steps a) or b) (or both, when multiple active ingredients are used), in step d) said suspension and solution are combined in such ratios as to obtain an overall formulation wherein the different components are present in the amounts defined above. The suspension thus obtained is then maintained under vigorous stirring (700-1200 rpm) for a time varying between 10 minutes and 1 hour, to favor optimal mixing and homogenization of all the components, obtaining a gel. Oxides of silicon, titanium, zirconium and aluminum are generally known in chemistry as silica, titania, zirconia and alumina; gels obtained in the present invention starting, as precursors, from silicon compounds only or titanium compounds only, will be referred to hereinafter as silica and titania gel, respectively; gels containing silica and titania as majority components will instead be referred to as silica-based or titania-based gels.

In step e), the obtained gel is allowed to rest in a closed container for a time of at least 10 days, and generally less than a month, to allow completion of the condensation and cross-linking reactions among the components used, leading to the achievement of the desired composition characteristics, mainly from the rheological point of view and in terms of active ingredient release properties. At the end of this period, the pH of the composition is adjusted to a value of between 4.3 and 5.5, compatible with the physiological pH of the skin; since the composition obtained after step e) generally has a pH of about 3.5, due to the presence of carboxylic acids and possibly HCI in case of silica-based compositions, this pH adjustment is performed by adding a base (for example, NaOH).

Optionally, essential oils or plant extracts, such as menthol, mallow extracts or tea plant extracts may then be added to the compositions thus obtained.

The process of the invention may moreover comprise a further optional step, d'), carried out between steps d) and e), consisting in the addition of a wetting agent whose function is keeping the appropriate amount of water in the gels; the wetting agent may be glycerol or, preferably, 1,3-propanediol.

Compared to the processes of the prior art, the process of the invention is innovative, versatile and easily scalable, and allows to obtain the final formulation in a few steps.

In the second aspect thereof, the invention relates to compositions in the form of a gel obtained by the process described above. These compositions comprise (as percentages by weight):
- water in an amount of 65 to 85%;
- one or more polysaccharides selected from chitosan, pectin, carrageenan, xyloglucan, an alginate, a starch, and mixtures thereof, in a total amount of 1 to 3%;
- one regulator of rheological properties selected from gum arabic, tragacanth gum, alginic acid and, preferably, guar gum, xanthan gum, or mixtures thereof, in a total amount of 0.5 to 1.5%;
- SiO₂, TiO₂ or mixtures thereof, and optionally ZrO₂, Al₂O₃ and ZnO, in a total amount of 3 to 6%;
- one or more alcohols in an amount of 5 to 10%;
- one or more cosmetic and/or medical compounds in an amount of 0.2 to 5%;
- salts, derived from neutralization of acids and bases used in the process, in an amount of 0.05 to 0.1%.

The compositions of the invention preferably further contain a wetting agent, usually 1,3-propanediol, in an amount of 3 to 5% by weight.

As stated in the process description, the suspensions and solutions prepared in steps a) and b) are preferably water-based only; the alcohols present in the compositions, in amounts of 5 to 10% by weight, derive from the hydrolysis of alkoxides or alkylalkoxides used as silica, titania, zirconia and alumina precursors, or are introduced in the compositions because the alkoxides employed as oxide precursors are sold commercially in the form of alcoholic solutions. These alcohols are not extracted from the compositions at the end of the preparation process (as could be done for example by evaporation, possibly under vacuum), because they act as preservatives for the same compositions.

The compositions of the invention, as well as the process for making them, have a series of advantages with respect to similar compositions of the prior art. In addition to using almost only raw materials of natural origin or produced from waste, the compositions are produced using a method that involves the addition, in a single step or at most two steps, of all the components in the reaction environment consisting of an aqueous base; this method allows to obtain compositions always having a gel or creamy texture, which allows easy spreadability and adherence on the skin, and results in a controlled release kinetics of active ingredients. Furthermore, by simply varying the type and amount of active ingredients, it is possible, using a single method and a single matrix, to create products for cosmetic use or directed to the treatment of different dermatological conditions, for example by formulating creams to be used in dermatology to counteract acne vulgaris, an anti-aging face cream, eye cream or anti-cellulite products. Another advantage of the gel compositions of the invention, experimentally confirmed by the inventors, is that they maximize the bioavailability of the active ingredients contained therein, thus allowing to reduce the number of applications and ensuring improved compliance by the end user. Finally, a further important peculiarity of the present invention is that the optimized formulation protocol allows to obtain gel compounds having good texture, that are homogeneous and stable without the use of any surfactant or dispersing agent (ingredients usually employed in commercial cosmetic formulations) and without the use of preservative molecules, improving for this reason also the compliance by the user.

The invention will be further illustrated by the following examples. In the experimental tests carried out, the following instruments and conditions were adopted:
- Anton Paar MCR 101 rheometer with flat-cone geometry, to assess the sample viscosity;
- Leica DM 1000 optical microscope, with 40x objective, used to evaluate the microscopic homogeneity of samples;
- Metrohm 691 pH meter to measure the pH of the gels; measurements taken at T = 25 °C.
- Franz's vertical diffusion cell, for the evaluation of the release properties of the active ingredients by the samples.

### EXAMPLE 1

This example refers to the preparation of a first anti-aging gel of the invention. The synthesis process is based on the preparation of a suspension containing chitosan and caffeine and a sol containing silica.

25 mL of acetic acid, 2% by volume (v/v) aqueous solution, and 100 mg of glycolic acid were introduced in a first container. 700 mg of caffeine were added to this solution, while maintaining the system under stirring. Once the caffeine was dissolved, 300 mg of chitosan were added to the solution. The acidic environment favors the biopolymer solubilization, thanks to protonation of the amino groups present. The final suspension thus obtained contains 2.3% by weight of caffeine.

Separately, a silica sol was prepared in a second container by introducing 3.5 mL of TEOS, 1.5 mL of MTES, and adding 0.1 mL of a 0.1 M HCI solution. 40 mg of azelaic acid, previously solubilized by vigorous stirring (400 rpm for 30 minutes) in 2 mL of water, were added to this suspension.

The suspension containing chitosan and the silica sol were then combined, still under vigorous stirring, until a homogeneous sol was obtained (this required about 20 minutes).

The sol thus obtained was allowed to rest for 15 days in a closed container, obtaining the formation of a gel, to which 1 mL of 1,3-propanediol was finally added as a wetting agent. After this period, 1 mL of a 9 M NaOH solution was added to the gel, obtaining a pH of 4.5.

The sample thus produced, containing 2% caffeine, 0.2% azelaic acid and 0.4% glycolic acid (weight percentages) is named sample 1.

This composition has an anti-aging action thanks to the simultaneous presence of caffeine, performing the effective antioxidant action, and glycolic acid, an alpha-hydroxy acid acting in synergy with caffeine by aiding its action, and maximizing the product effectiveness in counteracting skin aging.

### EXAMPLE 2

This example refers to the preparation of a second anti-aging gel of the invention.

500 mg of chitosan and 150 mg of guar gum, acting as rheological regulator, were dissolved in a first container, containing 40 mL of acetic acid, 2% v/v aqueous solution; the solubilization of the solid components was favored by magnetic stirring at a speed of 1200 rpm, and required about 30 minutes. After complete solubilization of the solid components, 150 mg of gallic acid were added to the suspension.

Simultaneously, 3.5 mL of TEOS, 1.5 mL of MTES, 2 mL of water and 0.2 mL of 0.1 M HCI were introduced into a second container, maintaining it under stirring at 400 rpm for 30 minutes.

At the end of this period, the contents of the two containers were combined, maintaining the system under stirring for 20 minutes; subsequently, 1 mL of 1,3-propanediol was added to the sol, as a wetting agent, to avoid excessive water evaporation. The sol thus obtained was allowed to rest for 15 days in a closed container, obtaining the formation of a homogeneous, clear and transparent gel. After this period, 0.7 mL of a 9 M NaOH solution was added to the gel, obtaining a pH of 4.5.

The sample thus obtained is named sample 2 and has a pH of 4.3.

### EXAMPLE 3

This example refers to the preparation of a third anti-aging gel of the invention.

750 mg of pectin and 150 mg of guar gum, acting as rheological regulator, were dissolved in a first container, containing 40 mL of acetic acid, 2% v/v aqueous solution; the solubilization of the solid components was favored by magnetic stirring at a speed of 1200 rpm, and required about 30 minutes. After complete solubilization of the solid components, 150 mg of gallic acid were added to the suspension.

Simultaneously, 3.5 mL of TEOS, 1.5 mL of MTES, 2 mL of water and 0.2 mL of 0.1 M HCI were introduced into a second container, maintaining it under stirring at 400 rpm for 30 minutes.

At the end of this period, the contents of the two containers were combined, maintaining the system under stirring for 20 minutes; subsequently, 1 mL of 1,3-propanediol was added to the sol, as a wetting agent, to avoid excessive water evaporation. The sol thus obtained was allowed to rest for 15 days in a closed container, obtaining the formation of a homogeneous, clear and transparent gel. After this period, 0.7 mL of a 9 M NaOH solution was added to the gel, obtaining a pH of 4.5.

The sample thus obtained is named sample 3 and has a pH of 4.3.

### EXAMPLE 4

This example relates to the assessment of the scalability of the procedure of the invention up to the industrial scale.

The procedure of Example 1 was repeated, increasing the amounts of each ingredient by ten times. In this case, magnetic stirring was replaced by mechanical stirring to be closer to the potential operating conditions in an industrial plant. In particular, polysaccharides and rheology regulators were solubilized using a magnetic stirrer at a speed of 5000 rpm, while the solution containing the TEOS/MTES mixture was maintained under stirring at 250 rpm for half an hour using a mechanical stirrer.

The sample thus obtained is named sample 4 and has a pH of 4.3.

### EXAMPLE 5

Samples 1-3 were subjected to measurements of viscosity, a fundamental parameter for cosmetic and dermatological compositions that must be spread on the skin.

Each sample was placed between the two surfaces of an Anton Paar MCR 101 rheometer with flat cone geometry, thermostated at 23 °C. The instrument lower surface is flat, while the upper surface is inclined by one degree (1°) with respect to the first. The samples were subjected to increasing stress through continuous rotation of the planes. The measured viscosity values are:
Sample 1: 2.5×10⁴ Pa·s;
Sample 2: 2.0×10⁴ Pa·s;
Sample 3: 2.0x10⁵ Pa·s.

All three samples have a viscosity in the pseudoplastic fluid regime, which is the optimal one for the intended application.

### EXAMPLE 6

Samples 1-3 were subjected to accelerated stability tests.

The first test consisted in verifying if the properties of the formulation remained unchanged when the sample is subjected to a mechanical stress, consisting in centrifugation at 4800 rpm for 30 minutes. The second, a stability test, consisted in the study of samples response to temperature changes, storing them in a refrigerator for 20 days at a temperature of 4 °C, and then for 20 days in an oven at 40 °C.

Stability was assessed by measuring the viscosity change before and after the two tests. All three samples passed the tests, showing an essentially unchanged viscosity (changes not exceeding ± 4%) with respect to the initial values; these tests suggest a stability of at least 6 months under normal conditions.

### EXAMPLE 7

In this example, release characteristics of active ingredients from the compositions of the invention are evaluated.

This measurement is performed through *in vitro* testing using a Franz vertical diffusion cell, a method commonly used to test semi-solid cosmetic formulations, according to the OECD 428 guideline available online at the link http://dx.doi.org/10.1787/20745788 (OECD Guidelines for the Testing of Chemicals, Section Health Effects, Test No. 428: Skin Absorption: In Vitro Method). The purpose of the test is to define the kinetics of dermal permeation (stratum corneum and epidermis) of active substances through isolated skin (*ex vivo* membranes). Fig. 1 shows a schematic and exploded view of the Franz cell; the cell, 10, consists of a receptor compartment, 11, which forms its lower part and contains the physiological solution; this solution is maintained at a constant temperature of 37 °C by means of an external jacket 12, surrounding the compartment 11 and connected to a thermostat (not shown in the figure) which circulates water at a constant temperature in the jacket through the inlet, 13, and outlet, 14, ducts; in the physiological solution there is a magnetic anchor, 15, which rotates slowly (200 rpm) and serves to keep the solution temperature homogeneous. The upper part of the cell is formed by the donor compartment, 16. Between the two compartments there is the membrane, 17, onto which the gel is homogeneously spread. The *ex vivo* membrane used were obtained from pig's ears, as they best mimic human skin from the mechanical, chemical-physical and microbiological point of view (H. Mollet, A. Grubenmann, Formulation Technology, Wiley-Vch Weinheim (2001), pp. 327-331). The membrane rests on a stainless steel screen (not shown in the figure). The donor compartment protects the gel from dust or external factors, prevents any evaporation of the volatile components of the suspension, and maintains the membrane and gel in contact with the physiological solution. At preset time intervals, the saline solution is withdrawn through the channel 18; the amount of solution withdrawn is replaced with an equal volume of fresh physiological solution. After filtration (45 µm PTFE solids filter), the withdrawn physiological solution is analyzed by UV-visible spectrophotometry or HPLC (high pressure liquid chromatography). At the end of the release tests, the membrane is extracted from the Franz cell, washed thoroughly to remove any gel residues, and treated to extract the amount of adsorbed active ingredients. For this purpose, the membrane is cut and subsequently left in contact with a suitable solvent (solvents are selected based on the type of active ingredient) for a time sufficient to ensure complete extraction of the active ingredients. The solution thus obtained is analyzed by UV-Vis or HPLC spectrophotometry.

In order to verify the characteristics of the samples of the invention, a test with identical methodology was also carried out on a commercial product sample with the same active ingredient content of sample 1; this reference sample is referred to as Ref. 1*. The release of active ingredients by samples 2 and 3 was instead compared with literature data on similar compositions, taken from the article "Skin delivery of antioxidant surfactants based on gallic acid and hydroxytyrosol", C. Alonso et al., Journal of Pharmacy and Pharmacology 67 (2014) 900-908; the comparison values for samples 1 and 2 are shown in the table as Ref. 2* and Ref. 3*.

Test results are shown in Table 1.

**Table 1**

| Sample | Amount of active ingredient absorbed | |
|---|---|---|
| | Absolute amount (mg) | Relative amount (%) |
| 1 | 3 | 58 |
| Ref. 1* | 1.5 | 30 |
| 2 | 400 | 40 |
| Ref. 2* | / | 9 |
| 3 | 215 | 25 |
| Ref. 3* | / | 9 |

As it can be seen from the data in the table, the active ingredients release rates of the samples of the invention are from about 2 to about 4 times higher than those of similar compositions of the prior art.

## Claims

1. Process for the preparation of a hybrid gel with three-dimensional network structure formed by inorganic components and naturally derived organic components, said gel containing one or more medical and/or cosmetic compounds, comprising the following steps:
a) in a first container, forming an aqueous or hydroalcoholic suspension containing between 1 and 4% by weight of at least one polysaccharide and between 0.5 and 1.5 by weight of at least one regulator of rheological properties;
b) in a second container, suspending in water an alkoxide or an alkylalkoxide of silicon, titanium or mixtures thereof, and optionally precursors of oxides of one or more metals selected from zirconium, aluminum, and zinc, in a total amount of between 50 and 80% by weight, and adding to the suspension thus obtained a hydrolysis catalyst in a molar ratio ranging from 0.001 to 0.01 with respect to the total moles of alkoxide or alkylalkoxide, obtaining a solution;
c) adding one or more medical and/or cosmetic compounds to the suspension obtained in step a) if said one or more compounds are lipophilic and/or to the solution obtained in step b) if said one or more compounds are hydrophilic, in an amount such that said medical and/or cosmetic compounds are from 0.2 to 5% by weight of the total weight of said suspension and solution;
d) combining the suspensions and solutions obtained in steps a), b) and c), and maintaining the resulting suspension under stirring for a time varying between 10 minutes and 1 hour, obtaining a gel;
e) allowing the gel obtained in step d) to rest in a closed container for a time of at least 10 days and bringing the pH of the same to a value between 4.3 and 5.5.

2. Process according to claim 1 comprising a further step d'), carried out between steps d) and e), consisting in the addition of a wetting agent to the gel obtained in step d).

3. Process according to one of claims 1 or 2, wherein the suspension of step a) is prepared using only water as a suspending agent.

4. Process according to any one of the preceding claims, wherein said polysaccharide is selected from chitosan having a molecular weight from 80 to 200 kDa, pectin having a molecular weight of about 100 kDa, carrageenan having a molecular weight from 100 to 500 kDa, xyloglucan having a molecular weight of about 600 kDa, an alginate having a molecular weight from 10 to 600 kDa, a starch having a molecular weight of about 200 kDa, and mixtures thereof.

5. Process according to any one of the preceding claims, wherein when said polysaccharide is chitosan or a mixture containing chitosan, a carboxylic acid selected from acetic acid and one of the acids used in the gel as medical and/or cosmetic compounds is added to the suspension of step a).

6. Process according to any one of the preceding claims, wherein said regulator of rheological properties is selected among gum arabic, tragacanth gum, alginic acid, guar gum, xanthan gum, and mixtures thereof.

7. Process according to any one of the preceding claims, wherein the solution of step b) is prepared using only water as a solvent.

8. Process according to any one of the preceding claims, wherein the alkoxide or alkylalkoxide used in step b) as precursor of Si and Ti has the general formula:
Rₓ-M-(OR')₄₋ₓ
wherein M is a metal selected from Si and Ti, R and R', equal or different, are C2-C4 alkyl radicals, and x is 0 or 1.

9. Process according to claim 8, wherein said Si or Ti alkoxide or alkylalkoxide is selected from tetraethoxysilane, Si(OCH₂CH₃)₄, methyltriethoxysilane, CH₃-Si(OCH₂CH₃)₃, and titanium tetraisopropoxide, Ti(OCH(CH₃)₂)₄.

10. Process according to any one of the preceding claims, wherein when the solution of step b) is prepared exclusively or with a major proportion of silicon alkoxides or alkylalkoxides, an acid is added to the solution.

11. Process according to any one of the preceding claims, wherein the precursors of the oxides of zirconium, aluminum, and zinc optionally added in step b) are selected from aluminum isopropoxide, zirconium isopropoxide, zirconium butoxide, zinc sulfate, zinc acetate, and zinc acetylacetonate.

12. Process according to any one of the preceding claims, wherein said medical and/or cosmetic compound is selected from glycolic acid, mandelic acid, gallic acid, azelaic acid, ferulic acid, and lipoic acid; caffeine; vitamin C and vitamin E; proanthocyanidins; polyphenols; and ellagic acid.

13. Process according to claim 12, wherein when the medical and/or cosmetic compound is a carboxylic acid, this is added in the form of an aqueous solution at a concentration ranging between 5 and 15% v/v.

14. Process according to any one of claims 2 to 13, wherein the wetting agent added in step d') is selected from glycerol and 1,3-propanediol.

15. Inorganic-organic hybrid gel obtained according to the process of any one of the preceding claims, comprising in percentage by weight:
- water in an amount of 65 to 85%;
- polysaccharides in an amount of 1 to 3%;
- regulators of rheological properties in an amount of 0.5 to 1.5%;
- SiO₂, TiO₂ or mixtures thereof, and optionally ZrO₂, Al₂O₃ and ZnO, in a total amount of 3 to 6%;
- one or more alcohols in an amount of 5 to 10%;
- medical and/or cosmetic compounds in an amount of 0.2 to 5%;
- salts, derived from neutralization of acids and bases used in the process, in an amount of 0.05 to 0.1%.

16. Inorganic-organic hybrid gel according to claim 15, further comprising at least a wetting agent in an amount of 3 to 5% by weight.

## Patentansprüche

1. Verfahren zur Herstellung eines Hybridgels mit dreidimensionaler Netzwerkstruktur, gebildet durch anorganische Komponenten und aus der Natur stammende organische Komponenten, wobei das Gel eine oder mehrere medizinische und/oder kosmetische Verbindungen enthält, umfassend die folgenden Schritte:
a) Bilden einer wässrigen oder hydroalkoholischen Suspension, die zwischen 1 und 4 Gew.% von mindestens einem Polysaccharid und zwischen 0,5 und 1,5 nach Gewicht von mindestens einem Regulator der rheologischen Eigenschaften enthält, in einem ersten Behälter;
b) Suspendieren in Wasser von einem Alkoxid oder einem Alkylalkoxid von Silicium, Titan oder Mischungen davon und gegebenenfalls Vorläufern von Oxiden von einem oder mehreren Metallen ausgewählt aus Zirconium, Aluminium und Zink in einer Gesamtmenge zwischen 50 und 80 Gew.% in einem zweiten Behälter, und Zufügen eines Hydrolysekatalysators in einem Molverhältnis im Bereich von 0,001 bis 0,01 in Bezug auf die Gesamtmol von Alkoxid oder Alkylalkoxid zu der so erhaltenen Suspension, wodurch eine Lösung erhalten wird;
c) Zufügen von einer oder mehreren medizinischen und/oder kosmetischen Verbindungen zu der in Schritt a) erhaltenen Suspension, falls die eine oder mehreren Verbindungen lipophil sind, und/oder zu der in Schritt b) erhaltenen Lösung, falls die eine oder mehreren Verbindungen hydrophil sind, in einer Menge, so dass die medizinischen und/oder kosmetischen Verbindungen 0,2 bis 5 Gew.% des Gesamtgewichts der Suspension und Lösung betragen;
d) Kombinieren der in den Schritten a), b) und c) erhaltenen Suspensionen und Lösungen, und Halten der resultierenden Suspension unter Rühren für eine Zeit, die zwischen 10 Minuten und 1 Stunde variiert, wodurch ein Gel erhalten wird;
e) Ruhen lassen des in Schritt d) erhaltenen Gels in einem geschlossenen Behälter für eine Zeit von mindestens 10 Tagen, und Bringen des pH-Werts des Gels auf einen Wert zwischen 4,3 und 5,5.

2. Verfahren nach Anspruch 1, umfassend einen weiteren Schritt d'), der zwischen den Schritten d) und e) durchgeführt wird, bestehend aus dem Zufügen eines Benetzungsmittels zu dem in Schritt d) erhaltenen Gel.

3. Verfahren nach einem der Ansprüche 1 oder 2, wobei die Suspension aus Schritt a) unter Verwendung von nur Wasser als Suspendiermittel hergestellt wird.

4. Verfahren nach einem der vorhergehenden Ansprüche, wobei das Polysaccharid ausgewählt ist aus Chitosan mit einem Molekulargewicht von 80 bis 200 kDa, Pektin mit einem Molekulargewicht von etwa 100 kDa, Karrageen mit einem Molekulargewicht von 100 bis 500 kDa, Xyloglukan mit einem Molekulargewicht von etwa 600 kDa, einem Alginat mit einem Molekulargewicht von 10 bis 600 kDa, einer Stärke mit einem Molekulargewicht von etwa 200 kDa und Mischungen davon.

5. Verfahren nach einem der vorhergehenden Ansprüche, wobei, wenn das Polysaccharid Chitosan oder eine Chitosan enthaltende Mischung ist, eine Carbonsäure ausgewählt aus Essigsäure und einer der Säuren, die in dem Gel als medizinische und/oder kosmetische Verbindungen verwendet wird, der Suspension aus Schritt a) zugefügt wird.

6. Verfahren nach einem der vorhergehenden Ansprüche, wobei der Regulator der rheologischen Eigenschaften ausgewählt ist aus Gummi arabicum, Tragantgummi, Alginsäure, Guargummi, Xanthangummi und Mischungen davon.

7. Verfahren nach einem der vorhergehenden Ansprüche, wobei die Lösung aus Schritt b) unter Verwendung von nur Wasser als Lösungsmittel hergestellt wird.

8. Verfahren nach einem der vorhergehenden Ansprüche, wobei das in Schritt b) als Vorläufer von Si und Ti verwendete Alkoxid oder Alkylalkoxid die folgende allgemeine Formel hat:
Rₓ-M-(OR')₄₋ₓ
wobei M ein Metall ausgewählt aus Si und Ti ist, R und R', die gleich oder verschieden sind, C2-C4-Alkylreste sind, und x 0 oder 1 ist.

9. Verfahren nach Anspruch 8, wobei das Si- oder Ti-Alkoxid oder -Alkylalkoxid ausgewählt ist aus Tetraethoxysilan, Si(OCH₂CH₃)₄, Methyltriethoxysilan, CH₃-Si(OCH₂CH₃)₃ und Titantetraisopropoxid, Ti(OCH(CH₃)₂)₄.

10. Verfahren nach einem der vorhergehenden Ansprüche, wobei, wenn die Lösung aus Schritt b) ausschließlich aus oder mit einem größeren Anteil an Siliciumalkoxiden oder -alkylalkoxiden hergestellt wird, der Lösung eine Säure zugefügt wird.

11. Verfahren nach einem der vorhergehenden Ansprüche, wobei die Vorläufer der Oxide von Zirconium, Aluminium und Zink, die gegebenenfalls in Schritt b) zugegeben werden, ausgewählt sind aus Aluminiumisopropoxid, Zirconiumisopropoxid, Zirconiumbutoxid, Zinksulfat, Zinkacetat und Zinkacetylacetonat.

12. Verfahren nach einem der vorhergehenden Ansprüche, wobei die medizinische und/oder kosmetische Verbindung ausgewählt ist aus Glykolsäure, Mandelsäure, Gallussäure, Azelainsäure, Ferulasäure und Liponsäure; Koffein; Vitamin C und Vitamin E; Proanthocyanidinen; Polyphenolen und Ellagsäure.

13. Verfahren nach Anspruch 12, wobei, wenn die medizinische und/oder kosmetische Verbindung eine Carbonsäure ist, diese in Form einer wässrigen Lösung in einer Konzentration im Bereich zwischen 5 und 15 % Vol./Vol. zugefügt wird.

14. Verfahren nach einem der Ansprüche 2 bis 13, wobei das in Schritt d') zugefügte Benetzungsmittel ausgewählt ist aus Glycerin und 1,3-Propandiol.

15. Anorganisch-organisches Hybridgel, das nach dem Verfahren gemäß einem der vorhergehenden Ansprüche erhalten wird, umfassend in Gewichtsprozent:
- Wasser in einer Menge von 65 bis 85 %;
- Polysaccharide in einer Menge von 1 bis 3 %;
- Regulator der rheologischen Eigenschaften in einer Menge von 0,5 bis 1,5 %;
- SiO₂, TiO₂ oder Mischungen davon und gegebenenfalls ZrO₂, Al₂O₃ und ZnO in einer Gesamtmenge von 3 bis 6 %;
- einen oder mehrere Alkohole in einer Menge von 5 bis 10 %;
- medizinische und/oder kosmetische Verbindungen in einer Menge von 0,2 bis 5 %;
- Salze, die aus der Neutralisierung von in dem Verfahren verwendeten Säuren und Basen stammen, in einer Menge von 0,05 bis 0,1 %.

16. Anorganisch-organisches Hybridgel nach Anspruch 15, des Weiteren umfassend mindestens ein Benetzungsmittel in einer Menge von 3 bis 5 Gew.%.

## Revendications

1. Procédé pour la préparation d'un gel hybride doté d'une structure de réseau tridimensionnel formée par des composants inorganiques et des composants organiques d'origine naturelle, ledit gel contenant un ou plusieurs composés médicaux et/ou cosmétiques, comprenant les étapes suivantes :
a) dans un premier récipient, formation d'une suspension aqueuse ou hydroalcoolique contenant entre 1 et 4 % en poids d'au moins un polysaccharide et entre 0,5 et 1,5 en poids d'au moins un régulateur de propriétés rhéologiques ;
b) dans un deuxième récipient, mise en suspension dans de l'eau d'un alcoxyde ou d'un alkylalcoxyde de silicium, de titane ou de mélanges correspondants, et éventuellement de précurseurs d'oxydes d'un ou plusieurs métaux choisis parmi le zirconium, l'aluminium et le zinc, en une quantité totale comprise entre 50 et 80 % en poids, et ajout à la suspension ainsi obtenue d'un catalyseur d'hydrolyse en un rapport molaire dans la plage de 0,001 à 0,01 par rapport au nombre total de moles d'alcoxyde ou d'alkylalcoxyde, obtenant une solution ;
c) ajout d'un ou plusieurs composés médicaux et/ou cosmétiques à la suspension obtenue dans l'étape a) si ledit ou lesdits composés sont lipophiles et/ou à la solution obtenue dans l'étape b) si ledit ou lesdits composés sont hydrophiles, en une quantité telle que lesdits composés médicaux et/ou cosmétiques sont présents de 0,2 à 5 % en poids du poids total de ladite suspension et solution ;
d) combinaison des suspensions et solutions obtenues dans les étapes a), b) et c), et maintien de la suspension résultante sous agitation pendant une durée variant entre 10 minutes et 1 heure, obtenant un gel ;
e) le fait de laisser le gel obtenu dans l'étape d) au repos dans un récipient fermé pendant une durée d'au moins 10 jours et le fait d'amener le pH de celui-ci à une valeur comprise entre 4,3 et 5,5.

2. Procédé selon la revendication 1 comprenant une étape supplémentaire d'), mise en œuvre entre les étapes d) et e), consistant en l'ajout d'un agent mouillant au gel obtenu dans l'étape d).

3. Procédé selon l'une des revendications 1 et 2, la suspension de l'étape a) étant préparée en utilisant seulement de l'eau en tant qu'agent de suspension.

4. Procédé selon l'une quelconque des revendications précédentes, ledit polysaccharide étant choisi parmi un chitosane possédant un poids moléculaire de 80 à 200 kDa, une pectine possédant un poids moléculaire d'environ 100 kDa, un carraghénane possédant un poids moléculaire de 100 à 500 kDa, un xyloglucane possédant un poids moléculaire d'environ 600 kDa, un alginate possédant un poids moléculaire de 10 à 600 kDa, un amidon possédant un poids moléculaire d'environ 200 kDa, et des mélanges correspondants.

5. Procédé selon l'une quelconque des revendications précédentes, lorsque ledit polysaccharide est un chitosane ou un mélange contenant un chitosane, un acide carboxylique choisi parmi l'acide acétique et l'un des acides utilisés dans le gel en tant que composés médicaux et/ou cosmétiques est ajouté à la suspension de l'étape a).

6. Procédé selon l'une quelconque des revendications précédentes, ledit régulateur de propriétés rhéologiques étant choisi parmi une gomme arabique, une gomme d'adragante, un acide alginique, une gomme de guar, une gomme xanthane et des mélanges correspondants.

7. Procédé selon l'une quelconque des revendications précédentes, la solution de l'étape b) étant préparée en utilisant seulement de l'eau en tant que solvant.

8. Procédé selon l'une quelconque des revendications précédentes, l'alcoxyde ou l'alkylalcoxyde utilisé dans l'étape b) en tant que précurseur de Si et Ti possédant la formule générale :
Rx-M-(OA')₄₋ₓ,
M étant un métal choisi parmi Si et Ti, R et R', identiques ou différents, étant des radicaux alkyles en C2-C4, et x étant 0 ou 1.

9. Procédé selon la revendication 8, ledit alcoxyde ou alkylalcoxyde de Si ou Ti 4 étant choisi parmi le tétraéthoxysilane, Si(OCH₂CH₃₄, le méthyltriéthoxysilane, CH₃Si(OCH₂CH₃)₃, et le tétraisopropoxyde de titane, Ti(OCH(CH₃)₂)₄.

10. Procédé selon l'une quelconque des revendications précédentes, dans lequel lorsque la solution de l'étape b) est préparée exclusivement ou avec une proportion majoritaire d'alcoxydes ou d'alkylalcoxydes de silicium, un acide est ajouté à la solution.

11. Procédé selon l'une quelconque des revendications précédentes, les précurseurs des oxydes de zirconium, d'aluminium et de zinc éventuellement ajoutés dans l'étape b) étant choisis parmi l'isopropoxyde d'aluminium, l'isopropoxyde de zirconium, le butoxyde de zirconium, le sulfate de zinc, l'acétate de zinc et l'acétylacétonate de zinc.

12. Procédé selon l'une quelconque des revendications précédentes, ledit composé médical et/ou cosmétiques étant choisi parmi l'acide glycolique, l'acide mandélique, l'acide gallique, l'acide azélaïque, l'acide férulique et l'acide lipoïque ; la caféine ; la vitamine C et la vitamine E ; des proanthocyanidines ; des polyphénols ; et l'acide ellagique.

13. Procédé selon la revendication 12, dans lequel lorsque le composé médical et/ou cosmétique est un acide carboxylique, il est ajouté sous la forme d'une solution aqueuse à une concentration dans la plage comprise entre 5 et 15 % v/v.

14. Procédé selon l'une quelconque des revendications 2 à 13, l'agent mouillant ajouté dans l'étape d') étant choisi parmi le glycérol et le 1,3-propanediol.

15. Gel hybride inorganique-organique obtenu selon le procédé selon l'une quelconque des revendications précédentes, comprenant en pourcentage en poids :
- de l'eau en une quantité de 65 à 85 % ;
- des polysaccharides en une quantité de 1 à 3 % ;
- des régulateurs de propriétés rhéologiques en une quantité de 0,5 à 1,5 % ;
- du SiO₂, du TiO₂ ou des mélanges correspondants, et éventuellement du ZrO₂, du Al₂O₃ et du ZnO, en une quantité totale de 3 à 6 % ;
- un ou plusieurs alcools en une quantité de 5 à 10 % ;
- des composés médicaux et/ou cosmétiques en une quantité de 0,2 à 5 % ;
- des sels, issus de la neutralisation d'acides et de bases utilisés dans le procédé, en une quantité de 0,05 à 1 %.

16. Gel hybride inorganique-organique selon la revendication 15, comprenant en outre au moins un agent mouillant en une quantité de 3 à 5 % en poids.
